(19) Europäisches Patentamt
European Patent Office
Office européen des brevets

(11) **EP 4 091 988 A1**

(12) **EUROPEAN PATENT APPLICATION**
published in accordance with Art. 153(4) EPC

(43) Date of publication:
**23.11.2022 Bulletin 2022/47**

(21) Application number: **20913842.9**

(22) Date of filing: **17.01.2020**

(51) International Patent Classification (IPC):
***C01B 33/12*** *(2006.01)*

(52) Cooperative Patent Classification (CPC):
**C01B 33/12**

(86) International application number:
**PCT/JP2020/001495**

(87) International publication number:
**WO 2021/144960 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Toyo Seikan Group Holdings, Ltd.**
**Shinagawa-ku**
**Tokyo 141-8627 (JP)**

(72) Inventors:
• **KIMURA, Mariko**
  **Yokohama-shi, Kanagawa 240-0062 (JP)**
• **OHASHI, Kazuaki**
  **Yokohama-shi, Kanagawa 240-0062 (JP)**
• **IKUTAME, Daisuke**
  **Yokohama-shi, Kanagawa 240-0062 (JP)**

(74) Representative: **Marks & Clerk LLP**
**15 Fetter Lane**
**London EC4A 1BW (GB)**

(54) **POROUS SILICA, DEODORANT AGENT, AND METHOD FOR PRODUCING DEODORANT AGENT**

(57) A porous silica having at least one metal X selected from the group consisting of Mn, Cu and Fe.

EP 4 091 988 A1

**Description**

Technical Field

The present invention relates to a porous silica, a deodorant agent, and a method for producing a deodorant agent.

Background Art

As deodorant agents, a wide variety of products are known. Among deodorant agents, there are constant needs for liquid deodorant agents depending on usage. In addition, various types of deodorant agents are known depending on odor to be eliminated. For example, there are also constant needs for deodorant agents for eliminating odor caused by sulfur-containing compounds (hereinafter referred to as sulfur-containing odor).

Regarding the above, for example, Patent Literature 1 (Japanese Patent Application Publication No. 2003-261425) discloses a deodorant treatment agent for hair treated with cysteamine, comprising one or two or more divalent metal ions. Cysteamine is a sulfur-containing compound.

Summary of Invention

Problems to be solved by the invention

The present inventors have been studying an enhancement in deodorization efficiency of liquid deodorant agents for eliminating sulfur-containing odor.

That is, an object of the present invention is to provide a liquid deodorant agent that is capable of efficiently eliminating sulfur-containing odor and a method for producing the same.

Means for solution of the problems

In order to solve the above problem, the present invention includes the following items.

[1] A porous silica doped with a metal X that is Mn or Cu.

[2] A porous silica for use in a liquid deodorant agent, comprising:

at least one metal X selected from the group consisting of Mn, Cu, and Fe.

[3] The porous silica according to [2], wherein

the porous silica is doped with the metal X.

[4] The porous silica according to any one of [1] to [3], further comprising:

a metal different from the metal X.

[5] The porous silica according to [4], wherein

the metal different from the metal X comprises a metal Y selected from the group consisting of Al and Zr.

[6] The porous silica according to [4] or [5], wherein

the metal different from the metal X comprises a metal Z selected from the group consisting of Co, Zn, Ag, and Ca.

[7] The porous silica according to [4], having a structure having a chemical composition represented by the following formula 1:

(formula 1) $SiO_2 \cdot {}_aXO_{b/2} \cdot cYO_{d/2} \cdot eZO_{f/2}$

wherein

X represents the metal X,

Y represents a metal Y selected from the group consisting of Al and Zr,

Z represents a metal Z selected from the group consisting of Co, Zn, Ag, and Ca, a represents a number that is larger than 0 and 0.1 or less,

c and e each represent a number that is 0 or more and 0.1 or less, and

b, d and f respectively represent valences of the metal X, the metal Y, and the metal Z.

[8] The porous silica according to any one of [1] to [7], wherein

a specific surface area of the porous silica is 500 $m^2$/g or more.

[9] A powder deodorant agent comprising:

the porous silica according to [1] to [8], wherein

the powder deodorant agent is a deodorant agent for permed hair.

[10] A liquid deodorant agent comprising:

the porous silica according to any one of [1] to [8]; and
a solvent.

[11] The liquid deodorant agent according to [10], wherein
pH of the liquid deodorant agent is 6 to 11.
[12] The liquid deodorant agent according to [10] or [11], wherein
the liquid deodorant agent is a deodorant agent for perm-treating hair.
[13] The liquid deodorant agent according to any one of [10] to [12], wherein
the liquid deodorant agent is a deodorant agent for sulfur-containing odor.
[14] The liquid deodorant agent according to any one of [10] to [13], further comprising:
a fragrance or a hair protection component.
[15] The liquid deodorant agent according to any one of [10] to [14], comprising:
water as the solvent.
[16] The liquid deodorant agent according to any one of [10] to [15], wherein
a content of the porous silica is 0.001 to 50% by mass.
[17] A method for producing a deodorant agent, comprising the steps of:

preparing a porous silica comprising at least one metal X selected from the group consisting of Mn, Cu, and Fe; and
mixing the porous silica with a solvent.

The present invention makes it possible to provide a liquid deodorant agent that is capable of efficiently eliminating sulfur-containing odor, and a method for producing the same.

Description of Embodiments

Hereinafter, an embodiment of the present invention will be described. A deodorant agent according to the present embodiment comprises: a solvent; and a porous silica. The porous silica is dispersed in the solvent, and comprises at least one metal X selected from the group consisting of Mn, Cu, and Fe. Employing such a configuration makes it possible to efficiently eliminate sulfur-containing odor.

1: Solvent

The solvent is not particularly limited as long as the porous silica is not dissolved but is dispersed in the solvent. Preferably, a liquid containing water as a main component (for example, 50% by mass or more) is used as the solvent.
The pH of the solvent is preferably 6 to 11, and more preferably 6 to 9. When the pH is 6 or more, no metal is eluted from the porous silica. When the pH is 11 or less, the porous silica is also not dissolved.
In addition, the solvent preferably has a low viscosity. When the solvent has a low viscosity, a sulfur-containing compound whose odor is to be eliminated is likely to diffuse to the insides of the pores of the porous silica, which makes it possible to shorten the time required for eliminating the odor. The viscosity of the solvent is, for example, 0.5 to 100 mPa/sec at 25°C.

2: Porous silica

The porous silica is dispersed in the solvent as described above. The content of the porous silica in the deodorant agent is, for example, 0.001 to 50% by mass, and preferably 0.1 to 10% by mass. When the content of the porous silica is 0.001% by mass or more, a sufficient deodorization effect can be achieved. When the content of the porous silica is 50% by mass or less, an effect that the fluidity can be maintained can be achieved.
The specific surface area of the porous silica is, for example, 500 $m^2$/g or more, preferably 800 to 2000 $m^2$/g, and more preferably 800 to 1600 $m^2$/g. When the specific surface area of the porous silica is 500 $m^2$/g or more, the area of contact between the metal X contained in the porous silica and the odor to be eliminated can be sufficiently secured, making it possible to achieve a high deodorization efficiency. In addition, when the specific surface area is 2000 $m^2$/g or less, the strength for maintaining the porous structure can also be secured.
The size of voids formed in the porous silica only has to be a size into and out of which the molecules of the solvent and the molecules of the sulfur-containing compound can move. For example, it is satisfactory that the diameter of the voids is 2 nm or more.

The metal X contained in the porous silica has a function of adsorbing sulfur-containing compounds. It is preferable that the porous silica be doped with the metal X. The term "doped" means that the metal X is incorporated in the position of a Si element in the $SiO_4$ skeleton of the silica.

Since the porous silica is doped with the metal X, the deodorization efficiency becomes higher than the case where the metal X is present in the form of granules without doping. It is considered that in the case where the porous silica is doped with the metal X, the metal X is present over the entire surfaces of the porous silica, and as a result, the area of contact between odor and the metal X becomes larger than the case where the metal X is present in the form of granules, so that high deodorization efficiency can be achieved.

In addition, in the case where the metal X is supported on the porous silica as a metallic salt soluble in a solvent, for example, it is considered that the metal X is eluted as metal ions in the solvent in a situation where the deodorant agent is used. In some cases, it is necessary to conduct high-cost treatments such as ion exchange in order to prevent metal ions from flowing as drain water. Hence, a cost for the treatment of drain water sometimes occurs. In contrast, in the case where the porous silica is doped with the metal X, the metal X is prevented from escaping from the porous silica, and it is thus possible to easily separate and collect the porous silica as a powder using a sedimentation apparatus, which reduces the cost for the treatment of drain water.

Furthermore, when the metal X is contained in the porous silica by doping, the color in the case where the metal X is present alone is reduced.

The content of the metal X in the porous silica is, for example, 0.01 to 10% by mass, and preferably 0.1 to 5% by mass. When the content of the metal X is 0.01% by mass or more, it is possible to achieve a sufficient deodorization effect. In addition, when the content of the metal X is 10% by mass or less, it is possible to easily synthesize the porous silica containing the metal X.

As the metal X, Mn or Cu is preferably used from the viewpoint of deodorant properties.

It is preferable that the porous silica contain a metal different from the metal X.

The metal different from the metal X includes, for example, a metal Y for suppressing hydrolysis of the porous silica. The metal Y includes, for example, a metal selected from the group consisting of Al and Zr. The porous silica is preferably doped with the metal Y.

When the porous silica is doped with the metal Y, the hydrothermal durability of the porous silica is enhanced. This is considered to be because when the silica is doped with the metal Y, hydrolysis of the siloxane skeleton of the silica is suppressed, so that the porous structure becomes unlikely to collapse.

The content of the metal Y in the porous silica is, for example, 0.01 to 10% by mass, and preferably 0.1 to 5% by mass. When the content of the metal Y is 0.1% by mass or more, it is possible to achieve an effect to maintain the specific surface area by storage over time due to the suppression of hydrolysis of the siloxane skeleton. When the content of the metal Y is 10% by mass or less, it is possible to achieve a high specific surface area of 500 $m^2$/g or more.

In addition, as a metal different from the metal X, the porous silica may contain a metal Z having a function of removing odor other than sulfur-containing odor. The metal Z includes, for example, at least one metal selected from the group consisting of Co, Zn, Ag, and Ca, and is preferably Co or the like. In the case where the porous silica contains Co or the like, it is expected that the porous silica decomposes aldehyde and eliminates fatty acid odor besides sulfur-containing odor, and a porous silica that is capable of eliminating many odors at once can be obtained.

The metal Z may be contained in the porous silica by "doping" or may be "supported on the porous silica as particles". The porous silica is preferably doped with the metal X.

Note that the term "supported on the porous silica as particles" means that the metal is supported on the porous silica without doping.

The content of the metal Z in the porous silica is, for example, 0.01 to 10% by mass, and preferably 0.1 to 5% by mass.

Specifically, it is preferable that the porous silica at least partially have a chemical structure represented by the following formula 1.

$$(\text{formula 1}): \quad SiO_2 \cdot_a XO_{b/2} \cdot cYO_{d/2} \cdot eZO_{f/2}$$

Note that in the formula 1, X represents the metal X.

Y represents the metal Y.
Z represents the metal Z.
a represents a number that is larger than 0 and 0.1 or less.
c and e each represent a number that is 0 or more and 0.1 or less.
b, d and f respectively represent valences of the metal X, the metal Y, and the metal Z.

Note that in order to dope the porous silica with the metal X, the metal Y, or the metal Z, a water-soluble metallic salt containing the metal X or the metal Y may be mixed with the porous silica or a precursor thereof in an aqueous solution during production of the porous silica. It can be confirmed that the porous silica is doped with a metal by measuring the chemical-bonding state through X-ray photoemission spectroscopy, Raman spectroscopy, or the like.

The deodorant agent according to the present embodiment achieves a function of removing sulfur-containing odor in water with the metal X contained by doping.

The sulfur-containing compound to be eliminated is not particularly limited as long as the sulfur-containing compound can be eliminated by the metal X contained by doping, but includes, for example, cysteamine, thioglycolic acid, cysteine, thiazolidine, methyl mercaptan, tert-butyl mercaptan, hydrogen sulfide, and the like.

Cysteamine, thioglycolic acid, cysteine, and the like are components contained in permanent agents, for example. Hence, the deodorant agent according to the present embodiment can be favorably used in order to remove odor such as cysteamine that is attached to hair subjected to permanent treatment.

In general, at the time of permanent treatment, hair is first wound on curlers. Subsequently, an agent called a permanent first agent is applied to the hair. The permanent first agent contains a reducing agent that cleaves cystine bonds of hair. Specifically, such a reducing agent includes cysteamine, thioglycolic acid, cysteine, and the like.

Once the cystine bonds are cleaved, the hair adapts to the shape wound on the curlers.

Thereafter, a permanent second agent is applied to the hair. The permanent second agent contains an oxidant. The cleaved cystine bonds are bonded again by the action of the oxidant. In this way, the shape of the hair is fixed to the shape wound on the curlers.

The deodorant agent according to the present embodiment can be used, for example, as a component to be blended in the above-described permanent second agent. Alternatively, the deodorant agent according to the present embodiment can be used also as an agent to be applied to hair separately from the permanent second agent. For example, the deodorant agent according to the present embodiment can be used by being blended in a conditioner, a hair spray, a hair wax, or the like which is applied to the hair after the permanent treatment.

The deodorant agent applied to the hair may be rinsed away or does not have to be rinsed away after exerting the deodorizing function.

The deodorant agent may contain a fragrance or a hair protection component. It is preferable that a fragrance or a hair protection component be present in the state of being contained in the porous silica. In the case where a fragrance or a hair protection component is contained, when the deodorant agent is applied to the hair and then left, the fragrance or the hair protection component is gradually released. In this way, it is also possible to achieve other functions such as elimination of body odor or scalp odor by the fragrance, and protection of the hair, in addition to the function of eliminating only sulfur-containing odor.

In order to make the porous silica contain a fragrance or a hair protection component, there is a method including: placing the porous silica into a solution in which these components are dispersed, followed by agitating; and then distilling away the solvent through vacuum drying, and the like.

The deodorant agent according to the present embodiment is not used only for deodorization of hair after permanent treatment, but can also be used for other usages. For example, raw garbage and the like contain sulfur-containing odor. For this reason, the deodorant agent according to the present embodiment can be favorably used also as a deodorization spray for raw garbage, and the like.

As described above, since the porous silica doped with the metal X is dispersed in the solvent, the deodorant agent according to the present embodiment is capable of efficiently eliminating sulfur-containing odor.

In particular, since the porous silica is used, the metal X, which expresses the deodorizing function, can be uniformly dispersed in the solvent, and as a result, the speed of chemical reaction between the metal X and a sulfur-containing compound can be improved.

In addition, the porous silica itself also has deodorizing performance for odor (acidic and basic odors) other than sulfur-containing odor. Hence, the deodorant agent according to the present embodiment is capable of eliminating odor other than sulfur-containing odor.

In addition, the porous silica is likely to be attached to hair. Hence, when the deodorant agent according to the present embodiment is applied to hair, the porous silica is attached to the hair. This makes it possible to more surely exert the deodorization action of the metal X.

3: Production method

The deodorant agent according to the present embodiment can be obtained by, after producing a porous silica, dispersing the porous silica in a solvent.

Note that the porous silica can be produced, for example, by a method comprising the following steps:

(A) a step of mixing a solvent, a surfactant, and a metal X-doping compound containing a metal X to prepare a surfactant solution;

(B) a step of adding a silica source to the surfactant solution to generate a micelle having the silica source aggregated on a surface thereof;

(C) a step of condensing the aggregated silica source; and

(D) a step of collecting and calcining the micelle after the step of condensing.

Hereinafter, each step will be described in detail.

(A): Preparation of surfactant solution

First, a surfactant and a metal X-doping compound are added to a solvent to prepare a surfactant solution. The surfactant solution is preferably agitated at room temperature or more and 200°C or less for 30 minutes or more and 10 hours or less. This causes the surfactant to form a micelle.

As the solvent, for example, water can be used. Alternatively, a mixture of water and an organic solvent such as ethanol or toluene may be used as the solvent.

The amount of the surfactant to be added is preferably 50 to 400 mmol/L, and more preferably 50 to 150 mmol/L.

Alternatively, the amount of the surfactant to be added is 0.01 to 5.0 mol, and preferably 0.05 to 1.0 mol, relative to 1 mol of the silica source to be added later.

The surfactant is not particularly limited, and any of cationic, anionic, and nonionic surfactants can be used.

The surfactant is preferably a neutral or cationic surfactant, and more preferably an alkyl ammonium salt. As the alkyl ammonium salt, an alkyl ammonium salt having 8 or more carbon atoms is preferable, and an alkyl ammonium salt having 12 to 18 carbon atoms is more preferable in view of industrial availability. The alkyl ammonium salt includes hexadecyltrimethylammonium chloride, cetyltrimethylammonium bromide, stearyltrimethylammonium bromide, cetyltrimethylammonium chloride, stearyltrimethylammonium chloride, dodecyltrimethylammonium bromide, octadecyltrimethylammonium bromide, dodecyltrimethylammonium chloride, octadecyltrimethylammonium chloride, didodecyldimethylammonium bromide, ditetradecyldimethylammonium bromide, didodecyldimethylammonium chloride, ditetradecyldimethylammonium chloride, and the like. One of these surfactants may be used alone, or two or more of these may be used in combination.

The metal X-doping compound is a substance serving as a source of supply of the metal X with which the porous silica is doped. As the metal X-doping compound, a water-soluble compound containing the metal X is preferably used. More preferably, a chloride, a nitrate, and a sulfate, and the like of the metal X are used.

For example, in the case where the metal X is iron, iron(III) chloride or the like is added as the metal X-doping compound.

For example, in the case where the metal X is copper, copper(II) nitrate or the like is added as the metal X-doping compound.

For example, in the case where the metal X is manganese, manganese(II) chloride or the like is added as the metal X-doping compound.

One metal X-doping compound may be used alone, or two or more metal X-doping compounds may be used in combination.

The amount of the metal X-doping compound to be added is, for example, 0.001 to 0.5 mol, and preferably 0.01 to 0.1 mol, relative to 1 mol of the silica source.

(B): Addition of silica source

Subsequently, the silica source is added to the surfactant solution.

The silica source is not particularly limited as long as the silica source serves as a raw material of the silica, but includes, for example, tetraethoxysilane, tetramethoxysilane, tetra-n-butoxysilane, sodium silicate, and the like. One of these silica sources may be used alone, or two or more of these may be used in combination. The silica source is preferably an alkoxy silane. The silica source is more preferably tetraethoxysilane. The organic functional group on the silicon atom is lost by hydrolysis, and thus does not affect the structure of the synthesized product. However, if the organic functional group is bulky, the speed of hydrolysis becomes low, and thus the synthesis time takes long time.

Note that in the case where sodium silicate is used alone or in combination as the silica source, the surfactant solution is heated and refluxed at 200°C or less for 20 to 2 hours.

The amount of the silica source to be added is not particularly limited, but is, for example, 0.2 to 1.8 mol/L. Alternatively, in the case where the solvent contains water, the concentration of the silica source is, for example, 0.001 to 0.05 mol relative to 1 mol of water.

(C): Condensation of silica source

Next, the silica source is condensed. Specifically, the pH of the solution is increased or decreased until the silica source is condensed. For example, the silica source can be condensed by adding a basic aqueous solution, followed by agitating. The agitation is conducted, for example, for 1 hour or more. By adding a basic aqueous solution, the silica source aggregated on the surface of the micelle is dehydrated and condensed to form walls of the silica.

The basic aqueous solution includes aqueous solutions of sodium hydroxide, sodium carbonate, ammonia, and the like. The basic aqueous solution is preferably an aqueous solution of sodium hydroxide. One of these basic aqueous solutions may be used alone or two or more of these may be used in combination. The basic aqueous solution is added such that the pH becomes preferably 8 to 14, and more preferably 9 to 11, immediately after the addition. By adding the basic aqueous solution, the dehydration and condensation reaction of the silica source is accelerated.

As a result, the surface tension of the condensed part increases, and the walls of the silica become spherical, and are turned into a form in which a large number of spheres are bonded, causing spinodal decomposition (phase separation). These structures are frozen by chemical cross-linking.

Note that the silica source has a characteristic to be condensed even when the pH is low. Hence, the silica source can also be condensed by adding an acidic aqueous solution but not a basic aqueous solution.

(D): Collecting and calcining of micelle

Subsequently, the micelle is collected as a precursor from the aqueous solution.

Particularly, when the silica source is condensed, the micelle precipitates. Then, the micelle is collected as a precursor by filtrating the precipitate. The precursor thus collected is dried. After the drying, the precursor is calcined to remove organic components contained in the precursor. That is, the surfactant of which the micelle is composed is removed. In this way, a porous silica having pores is formed. Note that the calcination is conducted at a decomposition temperature of the surfactant, or more. The calcination temperature is, for example, 400 to 600°C.

The porous silica obtained by the above-described method has been doped with the metal X derived from the metal X-doping compound. That is, the porous silica doped with the metal X is obtained.

Note that in the above-described method, an example in which the metal X-doping compound is added in step (A) is described. However, the metal X-doping compound does not necessarily have to be added in step (A), and may be added into the solution at any stage before the calcination in step (D). As long as the aggregated silica source and the metal X-doping compound are mixed in the solution, the metal X derived from the metal X-doping compound is incorporated into the silica source, so that the porous silica doped with the metal X is obtained.

In addition, in the case of producing a porous silica that contains a metal Y and/or a metal Z in addition to the metal X as the porous silica, a metal Y-doping compound or a metal Z-supplying compound may be added at a stage before step (D).

The metal Y-doping compound and the metal Z-supplying compound are substances serving as the sources of supply of the metal Y and the metal Z, respectively. As the metal Y-doping compound and the metal Z-supplying compound, a water-soluble compound containing the metal Y or Z is preferably used. More preferably, a chloride, a nitrate, a sulfate, and the like of the metal Y or the metal Z are used.

For example, in the case where the metal Y is aluminum, aluminum chloride or the like is added as the metal Y-doping compound.

For example, in the case where the metal Y is Zr, zirconium oxychloride or the like is added as the metal Y-doping compound.

For example, in the case where the metal Z is cobalt, cobalt nitrate or the like is added as the metal Z-supplying compound.

For example, in the case where the metal Z is Zn, zinc nitrate or the like is added as the metal Z-supplying compound.

For example, in the case where the metal Z is Ag, silver nitrate or the like is added as the metal Z-supplying compound.

For example, in the case where the metal Z is Ca, calcium carbonate or the like is added as the metal Z-supplying compound.

The metal Y-doping compound and the metal Z-supplying compound may be used alone, or two or more of them may be used in combination.

The amounts of the metal Y-doping compound and the metal Z-supplying compound to be added are each, for example, 0.001 to 0.5 mol, and preferably 0.01 mol to 0.1 mol, relative to 1 mol of the silica source.

(Examples)

Hereinafter, Examples of the present invention will be described. However, the present invention is not construed as being limited to these Examples.

Deodorant agents according to Examples 1 to 8 and Comparative Examples 1 to 6 were prepared. For each obtained deodorant agent, the specific surface area, the metal content, and the amount of cysteamine adsorbed were measured. Preparation methods and measuring methods for each of Examples and Comparative Examples are as described below.

Example 1

Hexadecyltrimethylammonium chloride as the surfactant, manganese chloride as the metal X-doping compound, and aluminum chloride as the metal Y-doping compound were added to water as the solvent, followed by agitating at 100°C for 1 hour. After the aqueous solution was cooled down to room temperature, tetraethoxysilane was added as the silica source, followed by agitating. Subsequently, sodium hydroxide was added as the condensation catalyst, followed by agitating. The amounts of the respective compounds added were set to the following amounts relative to 1 mol of tetraethoxysilane.

Surfactant (hexadecyltrimethylammonium chloride): 0.225 mol
Metal X-doping compound (manganese chloride): 0.0118 mol
Metal Y-doping compound (aluminum chloride): 0.0482 mol
Water: 125 mol
Sodium hydroxide: 0.195 mol

A solid product was separated by filtration from the suspension thus obtained, and was dried, followed by calcining to remove organic components to obtain a porous silica. This porous silica was obtained as a deodorant agent according to Example 1.

Example 2

Before the silica source was added, cobalt nitrate was further added as the metal Z-supplying compound. The amounts of the respective compounds added were set to the following amounts relative to 1 mol of tetraethoxysilane.

Surfactant (hexadecyltrimethylammonium chloride): 0.225 mol
Metal X-doping compound (manganese chloride): 0.0118 mol
Metal Y-doping compound (aluminum chloride): 0.0241 mol
Metal Z-supplying compound (cobalt nitrate): 0.011 mol
Water: 125 mol
Sodium hydroxide: 0.195 mol

A deodorant agent according to Example 2 was prepared in the same manner as in Example 1 except for the above point.

Example 3

Copper nitrate was used as the metal X-doping compound. The amounts of the respective compounds added were set to the following amounts relative to 1 mol of tetraethoxysilane.

Surfactant (hexadecyltrimethylammonium chloride): 0.225 mol
Metal X-doping compound (copper nitrate): 0.0204 mol
Metal Y-doping compound (aluminum chloride): 0.0482 mol
Water: 125 mol
Sodium hydroxide: 0.195 mol

A deodorant agent according to Example 3 was prepared in the same manner as in Example 1 except for the above point.

Example 4

Copper nitrate was used as the metal X-doping compound. Aluminum chloride and zirconium oxychloride were used as the metal Y-doping compounds. Cobalt nitrate was added as the metal Z-supplying compound. The amounts of the respective compounds added were set to the following amounts relative to 1 mol of tetraethoxysilane.

Surfactant (hexadecyltrimethylammonium chloride): 0.225 mol
Metal X-doping compound (copper nitrate): 0.0102 mol
Metal Y-doping compound (aluminum chloride): 0.0478 mol
Metal Y-doping compound (zirconium oxychloride): 0.021 mol
Metal Z-supplying compound (cobalt nitrate): 0.0116 mol
Water: 125 mol
Sodium hydroxide: 0.282 mol

A deodorant agent according to Example 4 was prepared in the same manner as in Example 1 except for the above point.

Example 5

Copper nitrate was used as the metal X-doping compound. Aluminum chloride and zirconium oxychloride were added as the metal Y-doping compound. The amounts of the respective compounds added were set to the following amounts relative to 1 mol of tetraethoxysilane.

Surfactant (hexadecyltrimethylammonium chloride): 0.225 mol
Metal X-doping compound (copper nitrate): 0.0102 mol
Metal Y-doping compound (aluminum chloride): 0.0498 mol
Metal Y-doping compound (zirconium oxychloride): 0.022 mol
Water: 125 mol
Sodium hydroxide: 0.282 mol

A deodorant agent according to Example 5 was prepared in the same manner as in Example 1 except for the above point.

Example 6

Iron chloride was added as the metal X-doping compound. The amounts of the respective compounds added were set to the following amounts relative to 1 mol of tetraethoxysilane.

Surfactant (hexadecyltrimethylammonium chloride): 0.225 mol

Metal X-doping compound (iron chloride): 0.0537 mol

Metal Y-doping compound (aluminum chloride): 0.0241 mol

Water: 125 mol

Sodium hydroxide: 0.195 mol

A deodorant agent according to Example 6 was prepared in the same manner as in Example 1 except for the above point.

Example 7

Oxine manganese was added as the metal X-doping compound. The metal Y-doping compound was not added. The amounts of the respective compounds added were set to the following amounts relative to 1 mol of tetraethoxysilane.

Surfactant (hexadecyltrimethylammonium chloride): 0.225 mol
Metal X-doping compound (oxine manganese): 0.0118 mol

Water: 125 mol
Sodium hydroxide: 0.195 mol

A deodorant agent according to Example 7 was prepared in the same manner as in Example 1 except for the above point.

Example 8

The surfactant or the metal Y-doping compound was not added. Copper nitrate was used as the metal X-doping compound. Nitric acid was used as the condensation catalyst. The amounts of the respective compounds added were set to the following amounts relative to 1 mol of tetraethoxysilane.

Metal X-doping compound (copper nitrate): 0.0102 mol
Water: 10 mol
Nitric acid: 0.1 mol

A deodorant agent according to Example 8 was prepared in the same manner as in Example 1 except for the above point.

Comparative Example 1

A manganese(IV) oxide powder (Wako 1st Grade, Catalogue Code 138-09675) produced by Wako Pure Chemical was prepared as Comparative Example 1.

Comparative Example 2

A powder obtained by dissolving cobalt chloride and manganese nitrate in water in a molar ratio of 1:1, followed by heating at 100°C to evaporate water, and then calcining at 570°C for 5 hours was prepared as Comparative Example 2.

Comparative Example 3

A copper nanoparticle reagent (Catalogue Code 794317) produced by Sigma-Aldrich was prepared as Comparative Example 3.

Comparative Example 4

A powder obtained by calcining iron chloride at 500°C for 5 hours was prepared as Comparative Example 4.

Comparative Example 5

Mesoporous silica MCM-41 (Catalogue Code 643645) produced by Sigma-Aldrich was prepared as Comparative Example 5.

Comparative Example 6

The metal X-doping compound was not added. Aluminum chloride and zirconium oxychloride were added as the metal Y-doping compounds. Cobalt nitrate was added as the metal Z-supplying compound. The amounts of the respective compounds added were set to the following amounts relative to 1 mol of tetraethoxysilane.

Surfactant (hexadecyltrimethylammonium chloride): 0.225 mol
Metal Y-doping compound (aluminum chloride): 0.0478 mol
Metal Y-doping compound (zirconium oxychloride): 0.035 mol
Metal Z-supplying compound (cobalt nitrate): 0.0231 mol
Water: 125 mol
Sodium hydroxide: 0.282 mol

A deodorant agent according to Comparative Example 6 was prepared in the same manner as in Example 1 except for the above point.

Method for measuring specific surface area

Measurement was conducted by a single-point method at liquid nitrogen temperature using FlowSorb II 2300 model manufactured by Micromeritics Instrument Corporation.

Method for measuring contents of metal X and metal Y

About 50 mg of the deodorant agent according to Example or Comparative Example was accurately weighed and dissolved in 4 ml of hydrochloric acid. Thereafter, the concentration of each metal (Mn, Cu, Fe, Al, Co, and Zr) in the aqueous solution was measured using ICP-OES manufactured by Thermo Scientific Corporation. It is considered that by treating with hydrochloric acid, all the metal components contained in the deodorant agent are dissolved in hydrochloric acid. In view of this, based on the result of measurement, the content of each metal present in the deodorant agent was calculated as the metal content.

Test for adsorbing cysteamine

First, 0.5896 g of cysteamine was weighed, and was diluted with ultra-pure water using a 10-ml volumetric flask to obtain a solution 1-1. The concentration of cysteamine in this solution 1-1 was 5.85 wt%, which was a concentration corresponding to an undiluted solution of a permanent agent.

In a laboratory centrifuge tube, 10 mg of the deodorant agent to be measured was weighed. Into the tube, 0.9 ml of water was added, and was well shaken to obtain a uniform dispersion liquid. To the dispersion liquid, 0.1 ml of the solution 1-1 was added and was well shaken for 30 seconds to be reacted. The concentration of cysteamine before the reaction was $7.64 \times 10^{-2}$ mol/L, and the initial amount of cysteamine was 5.89 mg. A centrifugation process was conducted for 90 seconds. The supernatant was promptly taken out, and the absorbance of the reaction liquid at 235 nm was measured.

From the measurement of a calibration curve, the concentration of cysteamine was within a range of $7.5 \times 10^{-4}$ mol/L to $1.2 \times 10^{-4}$ mol/L, and it was confirmed that a proportional relationship was established between the absorbance at 235 nm and the concentration of cysteamine. In view of this, the reaction liquid was diluted as appropriate so as to fall within this concentration range using an auto pipette, and absorbance at 235 nm was measured. From the result, the residual concentration of cysteamine was calculated in accordance with the following formula 1. In addition, the value of adsorption rate was calculated in accordance with the following formula 2.

(Formula 1) The residual concentration=a constant×the absorbance at 235 nm×the dilution factor

(Formula 2) The adsorption rate (%)=(the initial concentration-the residual concentration)/the initial concentration×100

Furthermore, the capacity and the like were calculated in accordance with the following formulae 3 to 5.

(Formula 3) The adsorbed amount (mg)=the adsorption rate (%)×the initial amount of cysteamine (mg)/100

(Formula 4) The amount of the metal X (mg)=the amount of the deodorant agent (mg)×the metallic amount of the metal X (wt%)/100

(Formula 5) The capacity (mg/mg of the metal X)=the adsorbed amount (mg)/the amount of the metal X (mg)

Note that the following absorbance measuring apparatus was used.

An absorbance measuring apparatus: Corona Absorbance Grating Microplate Reader SH-1000 (manufactured by CORONA ELECTRICS Co., Ltd.), plates: UV Flat Bottom Microtite (registered trademark) Plates (manufactured by Thermo), measurement range: 200 to 600 nm, response: 3

Results of measurement are shown in Table 1 and Table 2.

As shown in Table 1 and Table 2, the deodorant agents according to Examples 1 to 7 had improved adsorption rates of cysteamine as compared with the deodorant agent according to Comparative Example 6. From this, it was found that it was possible to improve the performance of adsorbing cysteamine by making the porous silica contain the metal X.

In addition, the deodorant agents according to Examples 1 to 8 had improved capacity for the adsorption of cysteamine as compared with the deodorant agents according to Comparative Examples 1 to 4 which did not use porous silica. From this, it was found that it was possible to improve the deodorization capacity of the metal X per unit mass by making the porous silica contain the metal X as compared with the case where the metal X was used as it was. That is, it was found that it was possible to achieve an equal or higher deodorization capacity using a smaller amount of of the metal X by making the porous silica contain the metal X.

In addition, the deodorant agents according to Examples 1 to 7 had higher adsorption rates of cysteamine than the deodorant agent according to Example 8. From this, it was found that with a higher specific surface area, it was possible to achieve a higher adsorption rate of cysteamine.

Furthermore, Examples 1 and 3 had higher abilities (rates and capacities) of adsorbing cysteamine than Example 6. From this, it was found that Mn and Cu are particularly preferable as the metal X.

Table 1

| Example | Specific surface area ($m^2/g$) | Metal X | | Metals Y and Z | | Adsorption of cysteamine | |
|---|---|---|---|---|---|---|---|
| | | Metal species | Metal amount wt% | Metal species | Metal amount wt% | Rate (%) | Capacity (mg/mg of the metal X) |
| 1 | 1176 | Mn | 0.63 | Al | 1.40 | 75 | 70 |
| 2 | 1061 | Mn | 0.58 | Co Al | 0.88 1.06 | 99 | 100 |
| 3 | 1409 | Cu | 2.09 | Al | 2.00 | 90 | 25 |
| 4 | 1200 | Cu | 0.86 | Co Al Zr | 0.66 1.73 1.88 | 91 | 35 |
| 5 | 1300 | Cu | 1.18 | Al Zr | 2.01 2.02 | 90 | 45 |
| 6 | 1383 | Fe | 3.32 | Al | 1.08 | 34 | 6 |
| 7 | 1200 | Mn | 1.07 | - | - | 51 | 28 |
| 8 | 103 | Cu | 0.36 | - | - | 6 | 9.6 |

Table 2

| Comparative Example | Specific surface area ($m^2/g$) | Metal X | | Metals Y and Z | | Adsorption of cysteamine | |
|---|---|---|---|---|---|---|---|
| | | Metal species | Metal amount (wt%) | Metal species | Metal amount (wt%) | Rate (%) | Capacity (mg/mg of the metal X) |
| 1 | less than 1 | Mn | 77 | - | - | 93 | 0.7 |
| 2 | less than 1 | Mn | 39 | Co | 36 | 76 | 1.1 |
| 3 | 19 | Cu | 80 | - | - | 23 | 0.1 |
| 4 | less than 1 | Fe | 70 | - | - | 7 | 0.1 |

(continued)

| Comparative Example | Specific surface area (m$^2$/g) | Metal X | | Metals Y and Z | | Adsorption of cysteamine | |
|---|---|---|---|---|---|---|---|
| | | Metal species | Metal amount (wt%) | Metal species | Metal amount (wt%) | Rate (%) | Capacity (mg/mg of the metal X) |
| 5 | 1000 | - | - | - | - | 8 | - |
| 6 | 1203 | - | - | Co<br>Al<br>Zr | 1.63<br>1.22<br>4 | 26 | - |

**Claims**

1. A porous silica doped with a metal X that is Mn or Cu.

2. A porous silica for use in a liquid deodorant agent, comprising:
   at least one metal X selected from the group consisting of Mn, Cu, and Fe.

3. The porous silica according to claim 2, wherein
   the porous silica is doped with the metal X.

4. The porous silica according to any one of claims 1 to 3, further comprising:
   a metal different from the metal X.

5. The porous silica according to claim 4, wherein
   the metal different from the metal X comprises a metal Y selected from the group consisting of Al and Zr.

6. The porous silica according to claim 4 or 5, wherein
   the metal different from the metal X comprises a metal Z selected from the group consisting of Co, Zn, Ag, and Ca.

7. The porous silica according to claim 4, having a structure having a chemical composition represented by the following formula 1:

   (formula 1) $SiO_2 \cdot_a XO_{b/2} \cdot cYO_{d/2} \cdot eZO_{f/2}$
   wherein
   X represents the metal X,
   Y represents a metal Y selected from the group consisting of Al and Zr,
   Z represents a metal Z selected from the group consisting of Co, Zn, Ag, and Ca,
   a represents a number that is larger than 0 and 0.1 or less,
   c and e each represent a number that is 0 or more and 0.1 or less, and
   b, d, and f respectively represent valences of the metal X, the metal Y, and the metal Z.

8. The porous silica according to any one of claims 1 to 7, wherein
   a specific surface area of the porous silica is 500 m$^2$/g or more.

9. A powder deodorant agent comprising:

   the porous silica according to any one of claims 1 to 8, wherein
   the powder deodorant agent is a deodorant agent for permed hair.

10. A liquid deodorant agent comprising:

    the porous silica according to any one of claims 1 to 8; and
    a solvent.

**11.** The liquid deodorant agent according to claim 10, wherein
pH of the liquid deodorant agent is 6 to 11.

**12.** The liquid deodorant agent according to claim 10 or 11, wherein
the liquid deodorant agent is a deodorant agent for perm-treating hair.

**13.** The liquid deodorant agent according to any one of claims 10 to 12, wherein
the liquid deodorant agent is a deodorant agent for sulfur-containing odor.

**14.** The liquid deodorant agent according to any one of claims 10 to 13, further comprising:
a fragrance or a hair protection component.

**15.** The liquid deodorant agent according to any one of claims 10 to 14, comprising:
water as the solvent.

**16.** The liquid deodorant agent according to any one of claims 10 to 15, wherein
a content of the porous silica is 0.001 to 50% by mass.

**17.** A method for producing a deodorant agent, comprising the steps of:

preparing a porous silica comprising at least one metal X selected from the group consisting of Mn, Cu, and Fe; and
mixing the porous silica with a solvent.

<table>
<tr><td colspan="2">**INTERNATIONAL SEARCH REPORT**</td><td>International application No.<br>PCT/JP2020/001495</td></tr>
</table>

A.  CLASSIFICATION OF SUBJECT MATTER
Int. Cl.  C01B33/12(2006.01)i
FI: C01B33/12 A

According to International Patent Classification (IPC) or to both national classification and IPC

B.  FIELDS SEARCHED

Minimum documentation searched (classification system followed by classification symbols)
Int. Cl.  C01B33/00-33/193, A61K8/00-8/99, A61Q1/00-90/00, B01J20/00-20/28, B01J20/30-20/34

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 2019-099399 A (TOYO SEIKAN GROUP HOLDINGS, LTD.) 24 June 2019, claims 1, 6-8, paragraphs [0033], [0037], example 4 | 1, 4-8<br>2-3, 9-17 |
| Y<br><br>A | JP 2017-132687 A (TOYO SEIKAN GROUP HOLDINGS, LTD.) 03 August 2017, paragraphs [0010]-[0017] | 2-3, 10-11, 13-17<br>1, 4-9, 12 |
| Y<br>A | JP 2004-131454 A (SHISEIDO CO., LTD.) 30 April 2004, claims 1-9 | 9, 12<br>1-8, 10-11, 13-17 |
| Y<br>A | WO 2002/049587 A1 (UNILEVER PLC) 27 June 2002, description, page 8, claims 1, 2 | 9, 12<br>1-8, 10-11, 13-17 |

☒  Further documents are listed in the continuation of Box C.　　☒  See patent family annex.

| | |
|---|---|
| *　　Special categories of cited documents:<br>"A"　document defining the general state of the art which is not considered to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search<br>01.04.2020 | Date of mailing of the international search report<br>14.04.2020 |
|---|---|
| Name and mailing address of the ISA/<br>　　Japan Patent Office<br>　　3-4-3, Kasumigaseki, Chiyoda-ku,<br>　　Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2020/001495

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2003-261425 A (NICCA CHEMICAL CO.) 16 September 2003, paragraphs [0002]-[0006] | 1-17 |

Form PCT/ISA/210 (continuation of second sheet) (January 2015)

International application No.

PCT/JP2020/001495

| Patent Documents referred to in the Report | Publication Date | Patent Family | Publication Date |
|---|---|---|---|
| JP 2019-099399 A | 24.06.2019 | (Family: none) | |
| JP 2017-132687 A | 03.08.2017 | US 2019/0001014 A1 entire text WO 2017/014186 A1 EP 3326972 A1 CN 107848818 A KR 10-2018-0022974 A | |
| JP 2004-131454 A | 30.04.2004 | (Family: none) | |
| WO 2002/049587 A1 | 27.06.2002 | AU 2367902 A | |
| JP 2003-261425 A | 16.09.2003 | (Family: none) | |

Form PCT/ISA/210 (patent family annex) (January 2015)

**EP 4 091 988 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003261425 A